# EUROPEAN PATENT SPECIFICATION

(11) **EP 2 870 962 B1**
(45) Date of publication and mention of the grant of the patent: **12.09.2018**
(21) Application number: 13812795.6
(22) Date of filing: 05.07.2013
(51) Int. Cl.: A61K 9/70, A61K 31/381, A61K 47/10, A61K 9/00, A61K 47/12, A61K 47/14

(54) **TRANSDERMALLY ABSORBABLE PREPARATION CONTAINING ROTIGOTINE**
TRANSDERMAL ABSORBIERBARES PRÄPARAT MIT ROTIGOTIN
PRÉPARATION ABSORBABLE PAR VOIE TRANSDERMIQUE CONTENANT DE LA ROTIGOTINE

(30) Priority: 05.07.2012 KR 20120073295
(43) Date of publication of application: 13.05.2015
(73) Proprietor: SK Chemicals Co., Ltd., Seongnam-si, Gyeonggi-do 463-400 (KR)
(72) Inventor: KIM, Hye-Min, Anyang-si Gyeonggi-do 430-010 (KR); HWANG, Yong-Youn, Suwon-si Gyeonggi-do 440-210 (KR); YOUN, Won-No, Seoul 151-015 (KR); PARK, Yeo-Jin, Seoul 131-200 (KR); OH, Joon-Gyo, Suwon-si Gyeonggi-do 440-709 (KR); IM, Jong-Seob, Suwon-si Gyeonggi-do 440-824 (KR); KIM, Hun-Taek, Seoul 137-951 (KR)
(74) Representative: Isarpatent
(86) International application number: PCT/KR2013/006001
(87) International publication number: WO 2014/007579

(56) References cited:
- EP-A1- 2 177 217
- WO-A1-03/092677
- WO-A1-2012/068783
- KR-A- 20030 025 941
- KR-A- 20040 104 697
- KR-A- 20050 038 007
- US-A1- 2005 079 206

## Description

### Technical Field

The present invention relates to a method for preventing the crystallization of rotigotine, and more particularly, to a method for preventing the precipitation of rotigotine crystals, the method including a step of mixing rotigotine with an anti-crystallizer, wherein the anti-crystallizer is oleic acid, wherein the rotigotine and the anti-crystallizer are mixed at a weight ratio of 10 : (6.62 to 20) to prevent the crystallization of rotigotine, and to a transdermally absorbable preparation containing rotigotine and anti-crystallizer.

### Background Art

Rotigotine, (-)-5,6,7,8-tetrahydro-6-[propyl-[2-(2-thienyl)ethyl]amino]-1-naphtahlenol, is used for the treatment of Parkinson's disease(PD) and restless legs syndrome (RLS), as a non-ergoline dopamine agonist.

This rotigotine has been commercially available in a patch dosage form. A patch-type transdermal composition is disclosed in U.S. Patent No. US 7413747 B. Also, one of the commercially available patches employs a silicone adhesive, which is disclosed in EP 1033978B. However, the commercially available patches cause the precipitation of rotigotine crystals in roomtemperature storage conditions, and thus it is difficult to secure the storage period necessary for commercial distribution of the commercially available patches. In order to solve the problem, patent WO 2011/076879 discloses a rotigotine transdermal composition with an added polymer, such as polyvinylpyrrolidone.

Rotigotine transdermal composition products on the market contain polyvinylpyrrolidone, but polyvinylpyrrolidone serves to increase the viscosity of a drug storage layer. As a result, the coating may not be uniform, and the uniform mixing of the drug storage layer is difficult. Thus, measures capable of preventing crystallization to solve the problems are being constantly researched.

US-A-2005079206 discloses an acrylic adhesive patch comprising rotigotine and oleic alcohol, and also discloses fatty acid esters of glycerol as suitable crystallization inhibitors.

### Detailed Description of the Invention

### Technical Problem

The present inventors, while researching methods for improving the storage stability of rotigotine patches, have found that rotigotine crystals are not formed when a patch is prepared by mixing rotigotine with fatty alcohols, fatty acids, or the like, and thus have completed the present invention.

Therefore, the present invention has been made in view of the above-mentioned problems, and an aspect of the present invention is to provide a method for preventing the crystallization of rotigotine, the method including a step of mixing rotigotine with an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

Another aspect of the present invention is to provide a transdermally absorbable preparation containing, as active ingredients, rotigotine and an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

Still another aspect of the present invention is to provide a method for preparing a transdermally absorbable preparation, the method including a step of mixing rotigotine with an anti-crystallizer, wherein the anti-crystallizer is oleic acid, and wherein the rotigotine and the anti-crystallizer are mixed at a weight ratio of 10 : (6.62 to 20) to prevent the crystallization of rotigotine.

Still another aspect of the present invention is to provide a transdermal therapeutic system including: a drug-containing adhesive layer including rotigotine, an adhesive, and an anti-crystallizer; and a substrate supporting the drug-containing adhesive layer, wherein the anti-crystallizer is oleic acid, wherein the drug-containing adhesive layer comprises the rotigotine, the anti-crystallizer, and the adhesive at a weight ratio of 10 : (6.62 to 20) : (95.73 to 96.4) to prevent the crystallization of rotigotine.

### Technical Solution

In accordance with an aspect of the present invention, there is provided a method for preventing the crystallization of rotigotine, the method including a step of mixing rotigotine with an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

In accordance with another aspect of the present invention, there is provided a transdermally absorbable preparation comprising, as active ingredients, rotigotine and an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

In accordance with still another aspect of the present invention, there is provided a method for preparing a transdermally absorbable preparation, the method comprising a step of mixing rotigotine with an anti-crystallizer, wherein the anti-crystallizer is oleic acid, and wherein the rotigotine and the anti-crystallizer are mixed at a weight ratio of 10 : (6.62 to 20) to prevent the crystallization of rotigotine.

In accordance with still another aspect of the present invention, there is provided a transdermal therapeutic system including: a drug-containing adhesive layer comprising rotigotine, an adhesive, and an anti-crystallizer; and a substrate for supporting the drug-containing adhesive layer, wherein the anti-crystallizer is oleic acid, wherein the drug-containing adhesive layer comprises the rotigotine, the anti-crystallizer, and the adhesive at a weight ratio of 10 : (6.62 to 20) : (95.73 to 96.4) to prevent the crystallization of rotigotine.

Hereinafter, the present invention will be described in detail.

The present invention provides a method for preventing the crystallization of rotigotine, the method including a step of mixing rotigotine with an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

The method for preventing the crystallization of rotigotine of the present invention is characterized by including a step of mixing rotigotine with an anti-crystallizer, wherein the anti-crystallizer is oleic acid, wherein the rotigotine and the anti-crystallizer are mixed at a weight ratio of 10 : (6.62 to 20) to prevent the crystallization of rotigotine.

Rotigotine ((-)-5,6,7,8-tetrahydro-6-[propyl-[2-(2-thienyl)ethyl]amino]-1-naphtahlenol) has a structure of chemical formula 1 below, and is used for the treatment of Parkinson's disease(PD) and restless legs syndrome(RLS), as a non-ergoline dopamine agonist.

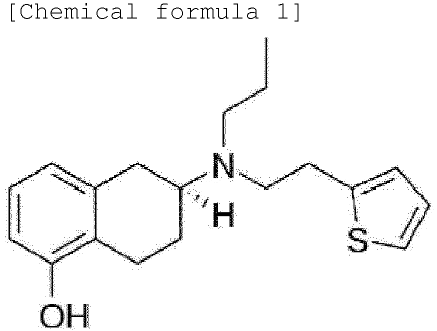

Described is an anti-crystallizer which can be used for suppressing the crystallization of rotigotine, and may be selected from the group consisting of fatty alcohols, fatty acids, fatty acid esters, fatty acid amides, and derivatives thereof. The fatty alcohols, fatty acids, fatty acid esters, and fatty acid amides of the anti-crystallizer may include at least one carbon atom, and may include no more than 40 carbon atoms. In addition, the materials of this group may not include a carbon-carbon double bond, or may include at least one carbon-carbon double bond. The anti-crystallizer may be selected from the group consisting of oleic acid, oleyl alcohol, caprylic acid (octanoic acid), and hexanoic acid.

The term "fatty alcohol" refers to any alcohol in which 8 to 22 carbon atoms are linked to each other in a chain shape. The fatty acids may be, for example, capryl alcohol, 2-ethyl hexanol, lauryl alcohol, tridecyl alcohol, myristyl alcohol, oleyl alcohol, and linoleyl alcohol, and may be preferably oleyl alcohol.

The term "fatty acid" refers to a long, aliphatic chain with a carboxylic group, and includes both saturated fatty acids and unsaturated fatty acids. The fatty acids may be, for example, myristoleic acid, palmitoleic acid, sapienic acid, oleic acid, and elaidic acid, and according to the present invention, the anti-crystallizer is oleic acid.

The term "fatty acid ester" refers to any ester resulting from the replacement of a hydrogen atom of fatty acid with an alkyl group, and the fatty acid esters may be, for example, isopropyl palmitate, isopropyl laurate, isooctylpalmitate, isooctyl stearate, lauryl stearate, butyl stearate, methyl n-caprylate, methyl n-caprate, methyl laurate, methyl stearate, and methyl oleate.

The term "fatty acid amide" refers to any amide resulting from the replacement of a hydrogen atom of fatty acid with an amide group, and the fatty acid amides may be, for example, oleamide, stearamide, erucamide, behenamide, N-oleylpalmitamide, and N-stearylerucamide.

The anti-crystallizer effectively blocks the crystallization of rotigotine, and enables the preparation of rotigotine-containing patches having high long-term storage stability. The fact that the crystallization of rotigotine is blocked when rotigotine is mixed with fatty alcohols, fatty acids, fatty acid esters, fatty acid amides, or the like is first presented in the specification of present application.

The amount of anti-crystallizer mixed with rotigotine is particularly limited, and the weight ratio of rotigotine and the anti-crystallizer is 10 : (6.62 to 20). Less than 0.1 parts by weight of anti-crystallizer added may cause the crystallization of rotigotine, and more than 40 parts by weight of anti-crystallizer added may lower the permeability of rotigotine into the skin, resulting in a smaller therapeutic effect.

The method of the present invention can be applied to various dosage forms comprising rotigotine as an active ingredient, and may be applied to preferably a patch, a liquid, an ointment, and the like.

Meanwhile, the present invention provides a transdermally absorbable preparation comprising rotigotine and an anti-crystallizer as active ingredients, wherein the anti-crystallizer is oleic acid, wherein the rotigotine and the anti-crystallizer are mixed at a weight ratio of 10 : (6.62 to 20) to prevent the crystallization of rotigotine.

The transdermally absorbable preparation of the present invention comprises rotigotine as an active drug ingredient, can conveniently administer the drug into the human body in a skin patch manner, and can constantly maintain a drug effect for a long time. In addition, the transdermally absorbable preparation of the present invention is not crystallized since it comprises an anti-crystallizer as an active ingredient, and thus can be stably stored for a long time.

The rotigotine and anti-crystallizer, which are the active ingredients of the transdermally absorbable preparation of the present invention, are as described as above.

Meanwhile, the transdermally absorbable preparation of the present invention may further comprise an adhesive in addition to the rotigotine and anti-crystallizer.

The adhesive allows the transdermally absorbable preparation of the present invention to be in continuous contact with the skin and thus facilitates the absorption of the active ingredients, and may include an acryl based adhesive, a rubber based adhesive, an ethylene-vinyl acetate based adhesive, a styrene based adhesive, and a silicone based adhesive. More preferably, the adhesive of the present invention may be a styrene based adhesive. The term "styrene based adhesive" refers to an adhesive comprising a styrene based bloc copolymer as a base material. The styrene based bloc copolymer may be at least one bloc copolymer selected from the group styrene-isoprene, styrenebutadiene, styrene-isoprene-styrene, styrene-butadienestyrene, styrene-isoprene-styrene-styrene, and styrenebutadiene-styrene-butadiene, but is not limited thereto. The adhesive of the present invention is preferably contained at a ratio of 40 to 500 parts by weight per 10 parts by weight of rotigotine.

The transdermally absorbable preparation of the present invention may further comprise a skin permeation promoter, a vehicle, an abirritant, or a carrier.

The skin permeation promoter is for promoting the permeation of a drug into the skin, and may include, for example, hydrophilic organic solvent, aprotic solvent, fatty acid, fatty alcohol, fatty acid ester, pyrrolidone, essential oil, surfactant, phospholipids, and the like.

The carrier contained in the transdermally absorbable preparation of the present invention is a biocompatible carrier, and may be, but is not limited to, a carrier for parenteral administration. The carrier for parenteral administration may include water, appropriate oil, a saline solution, aqueous glucose, glycol, and the like. For other pharmaceutically acceptable carriers, one disclosed in the following document may be referenced (Remington's Pharmaceutical Sciences, 19th ed., Mack Publishing Company, Easton, PA, 1995). The concentration of the biocompatible carrier may be, but is not limited to, about 2 to 70 wt%.

The transdermally absorbable preparation according to the present invention may be formulated into a patch, a liquid, an ointment, or the like, and may be formulated into, preferably a patch.

Further, the present invention provides a method for preparing a transdermally absorbable preparation, the method comprising a step of mixing rotigotine with an anti-crystallizer, wherein the anti-crystallizer is oleic acid, and wherein the rotigotine and the anti-crystallizer are mixed at a weight ratio of 10 : (6.62 to 20) to prevent the crystallization of rotigotine.

The method of the present invention is characterized by including a step of mixing rotigotine with an anti-crystallizer, wherein the anti-crystallizer is oleic acid, and wherein the rotigotine and the anti-crystallizer are mixed at a weight ratio of 10 : (6.62 to 20) to prevent the crystallization of rotigotine. The rotigotine and anti-crystallizer are as described above.

Further, the preparing method of the present invention may include a step of mixing an adhesive with a mixture of rotigotine and the anti-crystallizer.

According to the preparing method of the present invention, the rotigotine crystals are not precipitated, and thus a transdermally absorbable preparation having significantly improved long-term stability can be prepared.

Meanwhile, the present invention provides a transdermal therapeutic system comprising: a drug-comprising adhesive layer including rotigotine, an adhesive, and an anti-crystallizer; and a substrate supporting the drug-containing adhesive layer, wherein the anti-crystallizer is oleic acid, wherein the drug-containing adhesive layer comprises the rotigotine, the anti-crystallizer, and the adhesive at a weight ratio of 10 : (6.62 to 20) : (95.73 to 96.4) to prevent the crystallization of rotigotine.

The transdermal therapeutic system of the present invention is characterized by including a drug-containing adhesive layer including rotigotine, an adhesive, and an anti-crystallizer; and a substrate supporting the drug-containing adhesive layer, wherein the anti-crystallizer is oleic acid, wherein the drug-containing adhesive layer comprises the rotigotine, the anti-crystallizer, and the adhesive at a weight ratio of 10 : (6.62 to 20) : (95.73 to 96.4) to prevent the crystallization of rotigotine, and has a therapeutic effect against Parkinson's disease and restless legs syndrome through the permeation of rotigotine into the skin.

The drug-containing adhesive layer of the present invention is characterized by including rotigotine as an active drug, an anti-crystallizer for preventing the precipitation of rotigotine crystals, and an adhesive. The rotigotine, anti-crystallizer, and adhesive of the present invention are as described as above.

In the drug therapeutic system of the present invention, the content of anti-crystallizer may be, but is particularly not limited to, preferably more than 0.1 wt% to no more than 37 wt% based on the total weight of the drug-containing adhesive layer. If no more than 0.1 wt% of anti-crystallizer is added, it may be highly likely that rotigotine will be crystallized within four weeks, and if more than 37 wt% of anti-crystallizer is added, it may lower the skin permeability of the rotigotine.

In the drug-containing adhesive layer of the present invention, the ratio of rotigotine, the anti-crystallizer, and the adhesive is particularly limited, and the weight ratio of the rotigotine, the anti-crystallizer, and the adhesive is 10 : (6.62 to 20) : (95.73 to 96.4).

In cases where the proportion of the adhesive is lower than 40 parts by weight, the adhesive strength deteriorates and thus the drug-containing adhesive layer does not adhere to the skin. In cases where the proportion of the adhesive is higher than 500 parts by weight, the drug-containing adhesive layer adheres to the skin too firmly, and thus when the drug delivery system is attached to and detached from the skin, the skin may be irritated and the drug release may be delayed.

The transdermal therapeutic system of the present invention is characterized by including a substrate for supporting the drug-containing adhesive layer. For the substrate, any substrate that can be used for the known patches or the like may be used, and any substrate which is thin and flexible, has no reactivity with the drug-containing adhesive layer, and has no reactivity with the skin, causing no allergic reaction, is preferably used as the substrate of the present invention. For example, a non-woven fabric, polyethylene terephthalate, soft polyvinylchloride, polyurethane, polyester, and a silicone coating film may be used as the substrate of the present invention. Preferably, a silicone coating film or polyethylene terephthalate (PET) may be used.

In the transdermal therapeutic system of the present invention, the content of rotigotine may vary depending on the age, body weight, and gender of a patient, the manner of administration, the health condition, and the degree of severity of a disease, and may be applied once a day or divided into multiple doses such that 0.5 to 100 mg of an active material can be administered.

As for one example of the present invention, the drug-containing adhesive layer was prepared by mixing rotigotine, ethanol, hexane, polyisobutylene, and an anti-crystallizer, coated on a silicone coating film, and then bonded to a PET film, thereby manufacturing a transdermal therapeutic system. The transdermal therapeutic system was tested with respect to stability thereof by being stored for two weeks or four weeks in accelerated test conditions. As a result, it was confirmed that rotigotine crystals were precipitated in a control group not containing anti-crystallizer, and the rotigotine crystals were not precipitated in the transdermal therapeutic system of the present invention.

As another example of the present invention, the stability of rotigotine according to the change in the content of the anti-crystallizer was measured by varying the content of the anti-crystallizer contained in the transdermal therapeutic system. As a result, it was confirmed that the rotigotine crystals were not precipitated within a range of 5.9 wt% or more to 15.9 wt% or less of the anti-crystallizer based on the total weight of the drug-containing adhesive layer. It can be seen that the weight ratio of rotigotine and the anti-crystallizer is 10 : (6.62 to 20).

### Advantageous Effects

As set forth above, the present invention provides a method for preventing the crystallization of rotigotine, a transdermally absorbable preparation comprising rotigotine and an anti-crystallizer as active ingredients, a method for preparing the same, and a transdermal therapeutic system including a drug-containing adhesive layer including an anti-crystallizer, rotigotine, and an adhesive, and a substrate. The method and system of the present invention prevent the precipitation of rotigotine, thereby increasing the long-term storage stability, and thus can be effectively applied to the manufacture of rotigotine-containing patches or the like.

### Brief Description of the Drawings

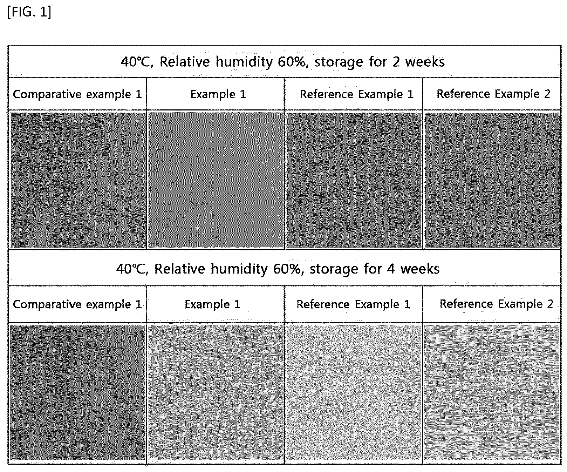

FIG. 1 illustrates images showing test results of an anti-crystallization effect according to the kind of anti-crystallizer of the present invention (Comparative example 1: anti-crystallizer non-addition group, Example 1: oleic acid addition group, Reference Example 1: caprylic acid addition group, and Reference Example 2: hexanoic acid addition group).

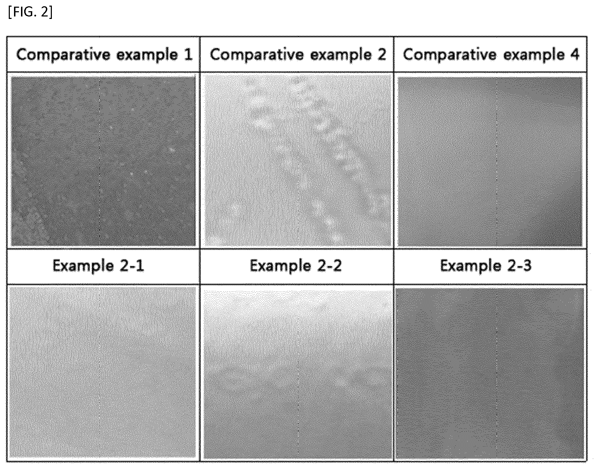

FIG. 2 illustrates images showing test results of an anti-crystallization effect according to the proportion of anti-crystallizer of the present invention (Comparative example 1: Anti-crystallizer non-addition group, Comparative example 2: 0.1% anti-crystallizer addition group, Comparative example 4: 0.6% oleic acid addition group, Example 2-1: 5.9% oleic acid addition group, Example 2-2: 8.6% oleic acid addition group, and Example 2-3: 15.9% oleic acid addition group).

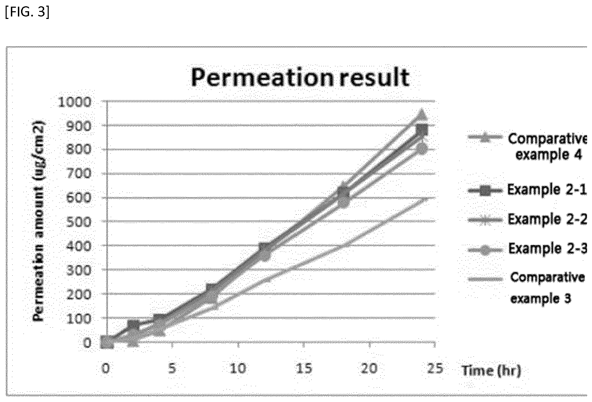

FIG. 3 illustrates images showing test results of the permeation into the human skin according to the proportion of anti-crystallizer (Comparative example 3: 37.0% anti-crystallizer addition group, Comparative example 4: 0.6% oleic acid addition group, Example 2-1: 5.9% oleic acid addition group, Example 2-2: 8.6% oleic acid addition group, and Example 2-3: 15.9% oleic acid addition group).

### Mode for Carrying Out the Invention

Hereinafter, the present invention will be described in detail with reference to the following examples,
However, the following examples are merely for illustrating the present invention, and are not intended to limit the scope of the present invention.

### <Example 1>

### Manufacture of patches containing anti-crystallizers and evaluation on crystallization therein

Rotigotine transdermal preparations with three kinds of anti-crystallizers were prepared following table 1 below.

**[Table 1]**

| Classification | Kind of anti-crystallizer |
|---|---|
| Comparative example 1 | Not added |
| Example 1 | Oleic acid |
| Reference Example 1 | Caprylic acid |
| Reference Example 2 | Hexanoic acid |

For comparative example 1, 300 mg of rotigotine was mixed with and completely dissolved in 600 mg of ethanol, and then 1 g of hexane was added thereto, thereby preparing a homogenous solution. For Example 1 and Reference Examples 1-2, 300 mg of rotigotine was mixed with and completely dissolved in 300 mg of ethanol and 300 mg of anti-crystallizers, and then 1 g of hexane was added thereto, thereby preparing homogenous solutions. 4.2 g of a styrene based adhesive was put into these solutions, followed by mixing for 10 minutes at 600 rpm or higher, thereby preparing transdermal absorbable preparations. The prepared transdermal absorbable preparations were allowed to stand at room temperature, thereby allowing bubbles to burst. After confirming the removal of bubbles, the mixtures without bubbles (transdermal absorbable preparations) were coated on silicone coating films such that rotigotine can be contained at 0.4-0.5 mg per unit area (1 cm²), and then polyester (PET) films were bonded thereto, thereby manufacturing patches. Each of the manufactured transdermal administration systems (patches) was molded into a circular shape of 10 cm², which was then put in a Petri dish, and then stored in accelerated test conditions of a temperature of 40 °C and a relative humidity of 60% (for 2 weeks or 4 weeks). The precipitation of crystals was observed by the naked eye and photographed by a digital camera.

As a result of testing, as shown in [FIG. 1], under accelerated test conditions of a temperature of 40 °C and a relative humidity of 60%, the precipitation of crystals was observed in comparative example 1 with no added anti-crystallizer, but the precipitation of crystals was not observed in Example 1 and Reference Examples 1-2 with added fatty acids in spite of storage for 2 weeks or 4 weeks. Therefore, it can be confirmed that the storage ability of the rotigotine transdermal preparation can be improved by adding an anti-crystallizer.

### <Example 2>

### Manufacture of patches for different proportions of anti-crystallizers and evaluation on crystallization therein

The crystallizing degree of rotigotine according to the proportion of anti-crystallizer was measured.

Patches were manufactured following ingredient contents listed on table 2. Specifically, by the same method as in example 1, a drug-containing matrix layer (rotigotine content: 0.4-0.5 mg/cm²), in which rotigotine, oleic acid, and a styrene based adhesive (Durotak 87-6911 (Henkel)) were coated on a silicone coating film, was formed, and then bonded to a support film of a polyester material, thereby manufacturing a patch coated with a transdermally absorbable preparation. The manufactured patch was molded into a circular shape of 10 cm², which was then put in a Petri dish, and then stored in accelerated test conditions of a temperature of 40°C and a relative humidity of 60% (for 2 weeks). The precipitation of crystals was observed by the naked eye and photographed by a digital camera.

**[Table 2]**

| Solid content (%) | Comparative example 1 | Comparative example 2 | Comparative example 3 | Comparative Example 4 | Example 2-1 | Example 2-2 | Example 2-3 |
|---|---|---|---|---|---|---|---|
| Rotigotine | 9.4 | 17.5 | 1.2 | 17.4 | 8.9 | 8.6 | 7.9 |
| Oleic acid | 0.0 | 0.1 | 37.0 | 0.6 | 5.9 | 8.6 | 15.9 |
| Adhesive | 90.6 | 82.4 | 61.7 | 82.0 | 85.2 | 82.8 | 76.2 |

In the following Table 2, rotigotine has been converted to provide "10" as a common basis for the solid content.

**[Table 2, converted]**

| Solid content (%) | Comparative example 1 | Comparative example 2 | Comparative example 3 | Comparative example 4 | Example 2-1 | Example 2-2 | Example 2-3 |
|---|---|---|---|---|---|---|---|
| Rotigotine | 10 | 10 | 10 | 10 | 10 | 10 | 10 |
| Oleic acid | 0.0 | 0.06 | 308.3 | 0.34 | 6.62 | 10 | 20 |
| Adhesive | 96.4 | 47.1 | 514.2 | 47.1 | 95.73 | 96.27 | 96.4 |

As can be seen from test results of FIG. 2, the crystals were precipitated while the transdermally absorbable preparation with no added anti-crystallizer (comparative example 1) was stored, and crystals of an active material were observed within 4 weeks after storage under conditions of a temperature of 40°C and a relative humidity of 60% in comparative example 2 in which oleic acid is contained in 0.1% based on the total solid content of the patch. However, crystals of an active material were not observed during the storage for 4 weeks or longer in the transdermally absorbable preparations with 0.6% or more of oleic acid (examples 2-1 to 2-3 in table 2). Therefore, the transdermally absorbable preparation containing an anti-crystallizer of the present invention does not cause crystallization even during the distribution at room temperature, thereby significantly improving the storage convenience.

### <Example 3>

### In vitro permeation test on patch according to proportion of anti-crystallizer

In vitro skin permeation test was conducted on the transdermally absorbable preparations prepared in example 2 using Franz cells (vertical diffusion cells, Hanson research). Each of the prepared transdermally absorbable preparations were molded into a size of 1 cm², which was then fixed on human cadaver skin, and then the test liquid inside the Franz cells, which passed through the skin from the transdermally absorbable preparation and diffused in the skin, was automatically collected every hour. The collected test liquid was pre-treated through centrifugation, and then was analyzed through high-performance chromatography.

The results are shown in FIG. 3, and confirmed the skin permeation of rotigotine according to the proportion of anti-crystallizer. The transdermally absorbable preparation, in which the proportion of oleic acid is 37% in the entire preparation (comparative example 3), showed relatively low rotigotine skin permeation The lower proportion of anti-crystallizer tends to show a relatively rapid rotigotine release pattern.

### Industrial Applicability

Accordingly, the present invention provides a method for preventing the crystallization of rotigotine, a transdermally absorbable preparation containing rotigotine and an anti-crystallizer, a method for preparing the same, and a transdermal therapeutic system including a drug-containing adhesive layer including an anti-crystallizer, rotigotine, and an adhesive, and a substrate. The method and system of the present invention prevent the precipitation of rotigotine, thereby increasing the long-term storage stability, and thus can be effectively applied to the manufacture of rotigotine-containing patches or the like. Therefore, the present invention is highly industrially applicable.

## Claims

1. A method for preventing the crystallization of rotigotine, the method comprising a step of mixing rotigotine with an anti-crystallizer,
wherein the anti-crystallizer is oleic acid,
wherein the rotigotine and the anti-crystallizer are mixed at a weight ratio of 10 : (6.62 to 20) to prevent the crystallization of rotigotine.

2. A transdermally absorbable preparation comprising, as active ingredients, rotigotine and an anti-crystallizer
wherein the anti-crystallizer is oleic acid,
wherein the rotigotine and the anti-crystallizer are mixed at a weight ratio of 10 : (6.62 to 20) to prevent the crystallization of rotigotine.

3. A method for preparing a transdermally absorbable preparation, the method comprising,
mixing the rotigotine with an anti-crystallizer,
wherein the anti-crystallizer is oleic acid, and
wherein the rotigotine and the anti-crystallizer are mixed at a weight ratio of 10 : (6.62 to 20) to prevent the crystallization of rotigotine.

4. A transdermal therapeutic system comprising:
a drug-containing adhesive layer comprising rotigotine, an adhesive, and an anti-crystallizer; and
a substrate for supporting the drug-containing adhesive layer,
wherein the anti-crystallizer is oleic acid,
wherein the drug-containing adhesive layer comprises the rotigotine, the anti-crystallizer, and the adhesive at a weight ratio of 10 : (6.62 to 20) : (95.73 to 96.4) to prevent the crystallization of rotigotine.

5. The system of claim 4, wherein the adhesive is selected from the group consisting of a silicone based adhesive, a rubber based adhesive, an acryl based adhesive, and an ethylene-vinyl acetate based adhesive.

6. The system of claim 5, wherein the rubber based adhesive is a styrene based adhesive.

7. The system of claim 4, wherein the anti-crystallizer is comprised in 5.9 wt% or more to 15.9 wt% or less based on the total weight of the drug-containing adhesive layer.

8. The method of claim 1 or 3, wherein the rotigotine is dissolved in ethanol, and anti-crystallizers and then hexane is added thereto.

## Patentansprüche

1. Verfahren, um die Kristallisation von Rotigotin zu verhindern, wobei das Verfahren den Schritt des Vermischens von Rotigotin mit einem Anti-Kristallisationsmittel umfasst,
wobei das Anti-Kristallisationsmittel Ölsäure ist,
wobei das Rotigotin und das Anti-Kristallisationsmittel in einem Gewichtsverhältnis von 10 : (6,62 bis 20) vermischt werden, um die Kristallisation von Rotigotin zu verhindern.

2. Transdermale absorbierbare Zubereitung, umfassend, als aktive Wirkstoffe, Rotigotin und ein Anti-Kristallisationsmittel,
wobei das Anti-Kristallisationsmittel Ölsäure ist,
wobei das Rotigotin und das Anti-Kristallisationsmittel in einem Gewichtsverhältnis von 10 : (6,62 bis 20) vermischt werden, um die Kristallisation von Rotigotin zu verhindern.

3. Verfahren zur Herstellung einer transdermalen absorbierbaren Zubereitung, wobei das Verfahren umfasst,
Vermischen des Rotigotins mit einem Anti-Kristallisationsmittel,
wobei das Anti-Kristallisationsmittel Ölsäure ist, und
wobei das Rotigotin und das Anti-Kristallisationsmittel in einem Gewichtsverhältnis von 10 : (6,62 bis 20) vermischt werden, um die Kristallisation von Rotigotin zu verhindern.

4. Transdermales therapeutisches System, umfassend:
eine Wirkstoff-enthaltende Klebstoffschicht, umfassend Rotigotin, einen Klebstoff und ein Anti-Kristallisationsmittel; und
ein Substrat, um die Wirkstoff-enthaltende Klebstoffschicht zu tragen,
wobei das Anti-Kristallisationsmittel Ölsäure ist,
wobei die Wirkstoff-enthaltende Klebstoffschicht das Rotigotin, das Anti-Kristallisationsmittel und den Klebstoff in einem Gewichtsverhältnis von 10 : (6,62 bis 20) : (95,73 bis 96,4) umfasst, um die Kristallisation von Rotigotin zu verhindern.

5. System nach Anspruch 4, wobei der Klebstoff ausgewählt ist aus der Gruppe, bestehend aus einem Silikon-basierten Klebstoff, einem Gummibasierten Klebstoff, einem Acryl-basierten Klebstoff, und einem EthylenVinylacetat-basierten Klebstoff.

6. System nach Anspruch 5, wobei der Gummi-basierte Klebstoff ein Styrolbasierter Klebstoff ist.

7. System nach Anspruch 4, wobei das Anti-Kristallisationsmittel in einer Menge von 5,9 Gew. % oder darüber bis 15,9 Gew. % oder darunter, bezogen auf das Gesamtgewicht der Wirkstoff-enthaltenden Klebstoffschicht, enthalten ist.

8. Verfahren nach Anspruch 1 oder 3, wobei das Rotigotin in Ethanol gelöst ist, und die Anti-Kristallisationsmittel und dann Hexan hierzu zugegeben werden.

## Revendications

1. Procédé de prévention de la cristallisation de la rotigotine, le procédé comprenant une étape de mélange de la rotigotine avec un anti-cristalliseur,
dans lequel l'anti-cristalliseur est l'acide oléique,
dans lequel la rotigotine et l'anti-cristalliseur sont mélangés à un rapport en poids de 10 : (6,62 à 20) pour prévenir la cristallisation de la rotigotine.

2. Préparation absorbable par voie transdermique comprenant, comme principes actifs, la rotigotine et un anti-critalliseur, dans lequel l'anti-cristalliseur est l'acide oléique,
dans lequel la rotigotine et l'anti-cristalliseur sont mélangés à un rapport en poids de 10 : (6,62 à 20) pour prévenir la cristallisation de la rotigotine.

3. Procédé de préparation d'une préparation absorbable par voie transdermique, le procédé comprenant,
le mélange de la rotigotine avec un anti-cristalliseur,
dans lequel l'anti-cristalliseur est l'acide oléique,
dans lequel la rotigotine et l'anti-cristalliseur sont mélangés à un rapport en poids de 10 : (6,62 à 20) pour prévenir la cristallisation de la rotigotine.

4. Système thérapeutique transdermique comprenant :
une couche adhésive contenant un médicament comprenant la rotigotine, un adhésif et un anti-cristalliseur ; et
un substrat pour supporter la couche adhésive contenant un médicament,
dans lequel l'anti-cristalliseur est l'acide oléique,
dans lequel la couche adhésive contenant un médicament comprend la rotigotine, l'anti-cristalliseur et l'adhésif à un rapport en poids de 10 : (6,62 à 20) : (95,73 à 96,4) pour prévenir la cristallisation de la rotigotine.

5. Système selon la revendication 4, dans lequel l'adhésif est choisi dans le groupe constitué par un adhésif à base de silicone, un adhésif à base de caoutchouc, un adhésif à base d'acryle et un adhésif à base d'éthylène-acétate de vinyle.

6. Système selon la revendication 5, dans lequel l'adhésif à base de caoutchouc est un adhésif à base de styrène.

7. Système selon la revendication 4, dans lequel l'anti-cristalliseur est compris dans 5,9 % en poids ou plus à 15,9 % en poids ou moins sur la base du poids total de la couche adhésive contenant le médicament.

8. Procédé selon la revendication 1 ou 3, dans lequel la rotigotine est dissoute dans de l'éthanol et des anti-cristalliseurs puis de l'hexane y est ajouté.
